Europäisches Patentamt

European Patent Office

Office européen des brevets

(19)

(11) Publication number: **0 102 207**
A2

(12)

# EUROPEAN PATENT APPLICATION

(21) Application number: 83304550.3

(22) Date of filing: 05.08.83

(51) Int. Cl.³: **C 12 N 11/00**
C 12 N 11/04, C 12 P 13/20

(30) Priority: 13.08.82 US 408048

(43) Date of publication of application:
07.03.84 Bulletin 84/10

(84) Designated Contracting States:
AT BE CH DE FR GB IT LI LU NL SE

(71) Applicant: THE BOARD OF TRUSTEES OF THE LELAND STANFORD JUNIOR UNIVERSITY
Encina 6-930, Stanford University
Stanford, California 94305(US)

(72) Inventor: Cohen, Stanley Norman
271 Gabarda Way
Portola Valley California 94025(US)

(72) Inventor: Robertson, Channing Rex
1089 Vernier Place
Stanford California 94305(US)

(72) Inventor: Inloes, Douglas Scott
818 South Brentwood Boulevard Apartment No. 3a
Clayton Missouri 63105(US)

(72) Inventor: Smith, William John
10F Escondido Village
Stanford California 94305(US)

(72) Inventor: Matin, Abdul
690 Coronado Avenue
Stanford California 94305(US)

(74) Representative: Allard, Susan Joyce et al,
BOULT, WADE & TENNANT 27 Furnival street
London EC4A 1PQ(GB)

(54) Method of immobilizing enzymes.

(57) A method is provided for contacting a substrate with an enzyme to produce an enzymatically modified product comprising the steps of: growing the cells which produce the enzyme in a volume confined by a membrane permeable to such substrate; contacting the cells with a lysing agent; and contacting the membrane with the substrate.

EP 0 102 207 A2

# METHOD OF IMMOBILIZING ENZYMES

The present invention is directed to a method for immobilizing enzymes for reaction with substrates to produce desirable enzymatically modified products. In particular, the present invention is directed to a method for immobilizing enzymes which are produced by cells confined in a restricted volume by a membrane.

This invention was made under Government support under Grants No. AI08619, GM26355 and GM27241 awarded by the National Institute of Health. The Government has certain rights in this invention.

In view of the recent advances in genetic engineering technology, there is an increasing interest in developing methods for utilizing microbial, animal and plant cells on a commercial scale to produce desirable biological products. In particular, there is a need to culture cells in a manner so that the enzymes and other cell products may be utilized to catalyze specific reactions.

The utilization of enzymes, however, has classically been a cumbersome and costly process since the typical approach is to grow the cells in a batch culture, to lyse the cells, to isolate and purify the desired enzyme by classical procedures, and then to immobilize the enzyme in a manner by which it may be efficiently contacted with the preferred substrate to produce the desired product. Such a multi-step process may not only be complex and expensive but it also suffers from the disadvantage that in isolating the enzyme, the enzyme is also removed from its natural cell environment. The stability and activity of an enzyme is often dependent on the presence of other cofactors, some of which need to be associated with specific membrane structures. Therefore, the use of enzymes on a commercial scale has always been complicated, on the one hand, by the need to isolate the enzyme and expose it to the preferred substrate in an efficient manner to optimize the yield of the desired product, while, on the other hand, meeting the need of the enzyme to coexist in an environment with its necessary cofactors and structural cell environment to optimize its enzymatic activity.

Preference to fulfill both needs have been attempted. For example Sato et al. (<u>Biochim. Biophys. Acta 570</u>; 179-86(1979)) disclose a process whereby cells are grown in batch culture and admixed with kappa-carrageenan gel and then precipitated to form small gelatinous beads. The beads are packed into a column and utilized as a packed bed biological reactor. However, this method suffers from the disadvantage that the solidified gel tightly entraps the cells making it difficult for the substrate and/or the enzyme contained within the cell to contact in an efficient manner. Moreover, whatever enzyme activity is achieved by this method is decreased by substrate/product concentration gradients which are dependent upon the distance of the cell beneath the surface of the bead. Furthermore, the process of solidifying the gel and transferring the beads into a column necessarily results in certain inefficiencies and waste of cell cultures by virtue of the physical transfers which are required.

Therefore there exists in the art a need to improve the efficiency in methods of utilizing enzymes in cells to catalyze reactions which produce a desirable product.

We have now developed a method for bringing enzymes and desirable substrates into contact in an improved manner to produce desirable biological products and a method for immobilizing enzymes and other cell products so that they may be utilized to catalyze specific chemical reactions.

The present invention provides a method of contacting a substrate with an enzyme to produce an enzymatically modified product, comprising the steps of a) growing cells which produce the desired enzyme whereby the cells are confined in a restricted volume by a membrane which is permeable to the substrate; b) contacting the cells with a lysing agent to permit the enzyme to be exposed to the substrate; and c) contacting the desired substrate with the membrane entrapped enzyme to affect the desired biocatalytic reaction.

In the accompanying figures:

FIGURE 1 is a schematic diagram of a hollow fiber microbiological reactor capable of being used in the practice of the present invention.

FIGURE 2 is a detailed schematic diagram of a hollow fiber membrane reactor.

The products which may be produced by the practice of the present invention include a variety of products which may be produced by the action of an enzyme on a specific substrate. The present invention may utilize various enzymes, including oxido-reductases which catalyze specific oxidation and/or reduction reactions, transferases which transfer functional groups from one molecular site to another, hydrolases which hydrolyze esters, glycosides, peptides, anhydrides and other types of bonds, lyases which catalyze addition to double bonds, isomerases, ligases and the like. The specific activity of the enzymes may be utilized to modify naturally occurring products to produce useful biological products or intermediates to other useful products. Enzymes may also be utilized to affect specific reaction in non-naturally occurring chemical compounds having reactive sites which are modifiable by enzymatic action.

According to the present invention a wide variety of microorganisms and cell types may be utilized provided such microorganisms or cell types contain or produce the enzyme of interest. These cells may be microorganisms such as bacteria, yeast, fungi, plants and the like, or proliferative cells derived from tissue, tumors, and hybridomas, and the like. These may be naturally occurring strains or cell lines, or those modified by conjugation or other known genetic engineering techniques.

The membranes utilized in the present invention include fibrous membranes having interstices in which a plurality of cells may be grown. Interstices should communicate with a porous wall which is permeable to the desired substrate. The substrate may then be contacted with the outside of the porous wall, preferably by a fluid stream, so that the substrate may diffuse or connectively flow through the pores and the product may reverse diffuse or connective flow through the porous wall back into the substrate stream and be carried away from the membrane. In this manner, the macroscopic environment of the enzyme and its cofactors remain relatively undisturbed by the flow and convection current of the substrate containing stream outside the porous wall.

A particularly preferred class of membranes comprises hollow fibers, which generally may be isotropic or asymmetric hollow fiber membranes. Such isotropic hollow fibers have a uniform tight mesh structure throughout the entire membrane whereas the asymmetric hollow fibers have a thin microporous inner lumen wall supported generally by a concentric thick macroporous matrix.

A preferred class of hollow fibers may be constructed of polymeric materials which may be hydrophobic, hydrophilic, positively or negatively charged, neutral or combinations thereof. Generally, the outside diameter of such fibers may be greater than 300 microns and less than 1500 microns. The inside diameter of such fibers may be at least 25% of the total diameter, however, the range of this dimension may be as high as approximately 90% of the total diameter. The wall thickness of hollow fibers may be greater than 15 microns and less than 500 microns. Examples of such fibers include XM-50, PM-30, PW-10 and VITAFIBER, all manufactured by Amicon Corporation; cellulose acetate and silicone polycarbonate hollow fibers made by Dow Chemical Corporation, Celgard made by Celanese Corporation and Biofiber 80-50 made by Bio-Rad Laboratories. Isotropic fibers are available from Ghia-Membrana.

Such membranes may be utilized in known microbiological reactor systems. For example a continuously stirred tank reactor known as a chemostat which accommodates both an inlet and outlet for continuous nutrient supply and product removal may be utilized.

The invention may be practiced by initial use of a medium for the growth of the cells followed by a gradual or substantially instantaneous change to the medium containing the substrate. The desired product may be retrieved either from the effluent liquid or, in the case of products sequestered intracellularly from either the disruption of cells washed out of the reactor or the batchwise disruption of all or part of the cell population.

Other types of microbiological reactors which are useful for the practice of the present invention include concentric airlift fermentors, tubular loop fermentors, tower fermentors, fluidized-bed fermentors, pressure cycle fermentors, semibatch fermentors, fermentors coupled to dialysis membranes, cells immobilized in or on a fiber matrix including hollow fiber membranes.

According to the present invention the cells are grown on the membrane to achieve a high cell density within the reactor system. Since a wide range of prokaryotic or eukaryotic cells may be utilized in the present invention, the specific growth environment used to grow the cells will depend upon the particular type of cell being cultured. In this respect the choice of growth environment is readily determined by those of ordinary skill in the art of growing the particular cell type or microorganism.

In the case of the preferred hollow fiber membranes, cell densities in the range of $10^9$-$10^{10}$ cells per ml may be obtained, when organisms such as yeast are used. In cultures of E. coli, cell densities as high as $10^{11}$-$10^{12}$ cells per ml are obtainable.

After the cells producing the enzyme of interest are grown on the membrane which confines the cell culture to a restricted volume, the cells are lysed in situ by contacting the cells with a lysing agent. The lysis may be accomplished by conventional cell lysing agents.

Conventional means may be utilized to lyse the cells according to the present invention such as, hypotonic lysis. Methods of cell lysis include use of conventional reagents such as magnesium chloride, SDS (sodium dodecyl sulfate) and sodium chloride. In some cases, the cell lysing agent also comprise the substrate which is to be modified by the enzyme. For example, as set forth below in the example, ammonium fumarate is both the lysing agent and the substrate for the aspartase which is present in the E. coli cells.

The substrates which may be modified by the action of enzymes, as described above, include both naturally occurring and non-naturally occurring chemical compounds. The substrate-containing medium is flowed through the lumen of the fiber, whereby the substrate may diffuse into the interstices containing the lysed cells and immobilized enzyme with its cofactors. The product of the enzyme-catalyzed reaction may be collected either by diffusion of the product through the porous membrane into the flow of the substrate-containing medium and collected or by flowing the substrate-containing medium through the reactor for a period of time and then removing the contents of the shell space within the reactor, including the products and cell materials and isolating the products therefrom.

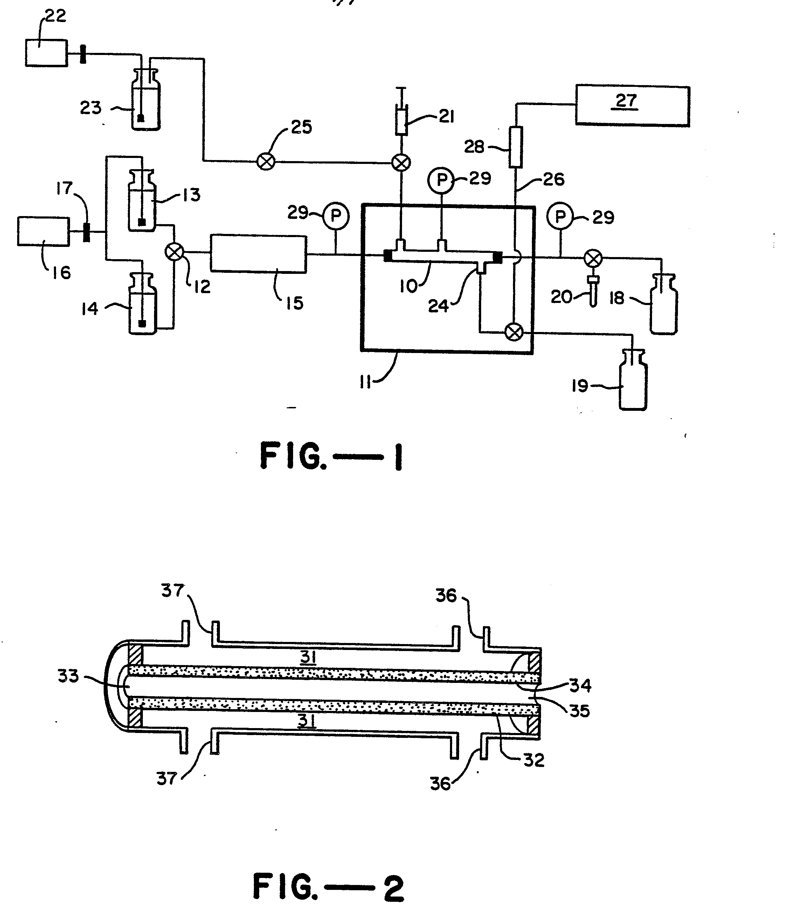

FIGURE 1 is a flow diagram of a microbiological reactor useful in accordance with the present invention. The microbiological reactor 10 containing hollow fiber membranes is located within gravity convection incubator 11 which maintains the environment of the reactor 10 at a constant temperature. By switching valve 12, either a growth medium contained in the vial 13 or a substrate and/or lysing medium in vial 14 may be pumped through the reactors through pump 15, which is preferably a peristaltic pump. The media in vial 13 and 14 may be kept under inert atmosphere, preferably helium, which is bubbled into the vial from inert gas containing tank 16 which is filtered through a sterile filter 17. The liquid effluents from reactor 10 may be collected into reservoirs 18 and 19. Reservoir 18 is a liquid effluent from the lumen of the hollow fibers and reservoir 19 collects the liquid effluent from the shell space surrounding the lumen. Small samples of the lumen effluent may be taken off through sample tube 20. The reactor 10 may be inoculated with appropriate cell cultures through inoculating syringe 21. Following inoculation the same port may be used for flushing sterile, humidified air from tank 22 through humidifier 23 into reactor 10. The shell space outlet port 24 is located below the level of reactor 10 to allow extraneous fluid to drain from the fiber bundle. If air flow into the shell space is required, a micrometric capillary valve 25 is located upstream of the reactor 10 to control the air flow rate through the shell space. Shell space fluid or air samples may be directed from the shell space effluent stream via line 26 into a gas or liquid chromatograph 27 through rotameter or pump 28. Various pressures may be monitored by manometers 29 located in various lines throughout the system.

The design of a reactor with a single hollow fiber is schematically represented in FIGURE 2. Cell innoculum is introduced into the shell space 31 of reactor 30 through an inoculation port, which may conveniently be 36 or 37. Chemotactic microorganisms are capable of moving through large diameter pores on the fiber surface into the macroporous wall matrix 32 following inoculation. This movement into the wall matrix may be enhanced by pulling a slight suction on the fiber lumen 33 if necessary to establish non-motile microbes. The introduction of at least one viable cell into a macropore will eventually lead to a dense cell packing as cells continue to grow and divide within the pore volume, essentially immobilizing and entrapping the actively growing cells within the wall matrix. Liquid phase nutrients are introduced into one end of the lumen and flow through the lumen and penetrate the lumen membrane 34 to enter the macroporous region containing the cells. During cell growth, metabolic products, excreted into the extra-cellular space within the fiber wall, diffuse or convect radially in the reverse direction across the membrane 34 into the fiber lumen where they are subsequently swept from the fiber through an exit port of the fiber lumen 35. When a substrate-containing medium or lysing medium is flowed through the lumen, the substrate or lysing agent diffuses across lumen membrane 34 into the cellular space 31. If required, continuous flushing of the shell space 31 with a gas stream through gas entry ports 36 will supply gaseous nutrients such as oxygen into the shell space, while simultaneously removing gaseous metabolic products produced during cell growth, such as carbon dioxide through gaseous exit ports 37. While FIGURE 2 has been described in connection with an asymmetric hollow fiber, the design of the reactor containing an isotropic hollow fiber is essentially the same as shown in FIGURE 2 except that the cells grow on the outer fiber surface 32 rather than within the fiber wall.

### Example

A reactor similar to that illustrated in FIGURE 1 containing a bundle of 150 asymmetric hollow-fiber membranes, was used to anaerobically culture E. coli. The reactor contained Dynel asymetric hollow fibers (Amicon Corporation) having dimensions of 200 micrometers I.D. x 300-500 micrometers O.D. The fibers were epoxied into a 5.7 centimeter by 7mm I.D. polycarbonate casing. Both ends of the reactor were sealed with epoxy leaving only the fiber lumens open to flow. The shell volume (total volume minus lumen volume) was 2.60 ml.

The shell space, was inoculated with aspartase synthesizing E. coli B (ATCC 1303). The culture was anaerobically grown to densities of $10^{12}$ cells per cubic centimeter within and around the porous walls of the asymmetric hollow fiber membrane on a glycerol-fumarate casamino acids medium (0.086 M fumarate, 0.078 M. glycerol, 3 g. casamino acids/liter, pH 6.5-7.0, 37°C).

Following the cell growth phase (usually 25-48 hrs. after inoculation). the nutrient feed was replaced by a concentrated (1 M) ammonium fumarate solution containing 1 mM $MgCl_2$ which lysed the cells and exposed the intracellular aspartase. With continuous flow of the fumarate solution through reactors and monitoring of fumarate concentration in the effluent by reading absorbance at 327 nm, volumetric productivity was observed to drop from 17 to 13 moles of aspartic acid/ hour/liter reactor volume accessible to the cells (i.e., total reactor volume minus lumen volume) and conversion efficiency of fumarate to aspartate was observed to drop from 90% to 70% at a fixed flow rate over a 6 week period. From calculations using a single fiber reactor, (conversion versus flow rate behavior of a single fiber reactor using a H10P10 polysulfone Amicon asymmetric single fiber, 965 micrometers O.D., 508 micrometers I.D., epoxied in 30.4 cm long x 1066 micrometer I.D. stainless steel tube), the volumetric productivity based on a total volume of a single fiber reactor was, at 95% conversion, about twice that reported for a gel-particle immobilized cell aspartase reactor according to Chibata, Biohim. Biophys. Acta. 570; 179-186 (1979).

-12 -

CLAIMS:

1. A method for contacting a substrate with an enzyme to produce an enzymatically modified product, comprising the steps of:

a. growing cells which produce the enzyme, the cells being confined in a restricted volume by a membrane permeable to the substrate;

b. contacting the cells with a lysing agent to expose the enzyme; and

c. contacting the enzyme with the substrate.

2. A method as claimed in Claim 1 wherein the cells are grown in a hollow fiber membrane.

3. A method as claimed in claim 2 wherein the membrane is an asymmetric hollow-fiber membrane.

4. A method as claimed in any one of claims 1 to 3 wherein the cells are grown in a hollow fiber membrane.

5. A method as claimed in claim 5 wherein the cells are bacteria.

6. A method according to claim 1 wherein the cells are proliferative eukaryotic cells derived from tissue.

7. A method as claimed in any one of the preceding claims wherein the substrate comprises the lysing agent.

8. A method as claimed in claim 7 wherein the enzyme is aspartase, the substrate comprises fumarate and the product is aspartate.

0102207

1/1

FIG.—1

FIG.—2